# EUROPÄISCHE PATENTSCHRIFT

(11) **EP 1 663 036 B1**
(45) Veröffentlichungstag und Bekanntmachung des Hinweises auf die Patenterteilung: **18.11.2009**
(21) Anmeldenummer: 03818609.4
(22) Anmeldetag: 18.09.2003
(51) Int. Cl.: A61B 17/74, A61B 17/72

(54) **VORRICHTUNG ZUR BEHANDLUNG VON FRAKTUREN DES FEMUR**
DEVICE FOR TREATING FEMORAL FRACTURES
DISPOSITIF DE TRAITEMENT DE FRACTURES FEMORALES

(43) Veröffentlichungstag der Anmeldung: 07.06.2006
(73) Patentinhaber: Synthes GmbH, 4436 Oberdorf (CH)
(72) Erfinder: FRIGG, Robert, CH-2544 Bettlach (CH); HATTLER, Eric, CH-4500 Solothurn (CH); WIDMER, Walter, CH-4515 Oberdorf (CH); BARRIOS, Elena, CH-2503 Biel (CH); KÜPPERS, Stephan, CH-4552 Derendingen (CH)
(74) Vertreter: Lusuardi, Werther
(86) Internationale Anmeldenummer: PCT/CH2003/000630
(87) Internationale Veröffentlichungsnummer: WO 2005/025435

(56) Entgegenhaltungen:
- WO-A-03/028567
- DE-U- 8 701 164
- US-A1- 2002 156 473
- US-B1- 6 423 066

## Beschreibung

Die Erfindung bezieht sich auf eine Vorrichtung zur Behandlung von Frakturen des Femur gemäss dem Oberbegriff des Patentanspruchs 1.

Es sind bereits Vorrichtungen bekannt, bei welchen eine Rotationssicherung des Femurkopfes mit einer einzigen Hüftschraube, d.h. einem longitudinalen Knochenfixationsmittel zu erreichen versucht wird. Aus der EP-B 0 441 577 ist beispielsweise eine solche Vorrichtung bekannt, welche eine die Hüftschraube gleitend aufnehmende Hülse aufweist, wobei die Hülse mittels einer von proximal in den Marknagel einführbaren Blockierschraube im Marknagel gegen Rotation gesichert werden kann. Der Schaft der Hüftschraube und die Bohrung der Hülse sind allerdings unrund ausgebildet, damit sich die Hüftschraube in der Hülse nicht drehen kann. Die Hüftschraube muss sich aber während des Einbringen in den Hüftkopf drehen können. Aus diesem Grund muss bei der Implantation zuerst die Hüftschraube eingesetzt werden und erst danach die Gleithülse. Im weiteren besteht die Gefahr, dass die Hüftschraube nach medial wandert, wenn nicht zusätzlich eine Kompressionsschraube verwendet wird. Ein weiterer Nachteil besteht darin, dass die Blockierschraube von oben (kranial) in den Marknagel eingeführt werden muss, was einen zusätzlichen Operationsschritt bedeutet. Schliesslich ist bei einer allfällig später notwendig werdenden Entfernung der Hüftschraube ein relativ grosser Eingriff notwendig, um in einem Schritt die von proximal in den Marknagel eingeschraubte Blockierschraube zu lösen, bevor die Hüftschraube entfernt werden kann.

Im weiteren ist aus der US-A 5,454,813 LAWES (Basis für den Oberbegriff des Anspruchs 1) ein Marknagel mit einer Hüftschraube und mit einer Gleithülse bekannt, bei welchem der Durchgang im Marknagel, das äussere und innere Profil der Gleithülse und der Schaft der Hüftschraube unrund ausgebildet sind. Die Gleithülse wirkt hier somit als Antirotationsmittel zwischen der Hüftschraube und dem Marknagel. Diese bekannte Vorrichtung weist die Nachteil auf, dass die vorgängig eingebrachte Hüftschraube während der Montage der Gleithülse wieder vorwärts- oder rückwärtsgedreht werden muss, bis sie so ausgerichtet ist, dass die unrunden Querschnitte am Schaft, des inneren und äusseren Profiles der Gleithülse und des Durchganges das Einführen der Gleithülse gestatten. Dies bedeutet eine zeitaufwendige Justierung für den Operateur. Ein weiterer Nachteil dieser bekannten Vorrichtung besteht darin, dass eine mediale Wanderung nur mit einem zusätzlichen Bauelement (Spannungseinsteller) verhindert werden kann.

Die obenstehende Diskussion des Standes der Technik erfolgt lediglich zur Erläuterung des Umfeldes der Erfindung und bedeutet nicht, dass der zitierte Stand der Technik zum Zeitpunkt dieser Anmeldung auch tatsächlich publiziert oder öffentlich bekannt war.

Hier will die Erfindung Abhilfe schaffen. Der Erfindung liegt das Problem zugrunde, eine Vorrichtung zur Behandlung von Knochenfrakturen, insbesondere von proximalen Femurfrakturen zu schaffen, welche während der Implantation keine aufwendigen Justiervorgänge für den Operateur erfordert und einfach blockier- und deblockierbare, formschlüssige Rotationsblockierung zwischen dem longitudinalen Knochenfixationselement (z.B. der Hüftschraube) und dem Marknagel erlaubt.

Die Erfindung löst die gestellte Aufgabe mit einer Vorrichtung zur Behandlung von Frakturen des Femur, welche die Merkmale des Anspruchs 1 aufweist.

Die durch die Erfindung erreichten Vorteile sind im wesentlichen darin zu sehen, dass dank der erfindungsgemässen Vorrichtung
- das Knochenfixationselement relativ zum Marknagel mittels der Blockiermittel bezüglich Rotation um die Längsachse des Knochenfixationselementes wahlweise blockierbar oder deblockierbar ist;
- die Blockiermittel von lateral blockier- respektive deblockierbar sind;
- vor der Montage der Blockiermittel wegen der Rotierbarkeit der Gleithülse auf dem Schaft des Knochenfixationselementes das Einführen der Gleithülse nach der Implantation des Knochenfixationselementes ohne langes Justieren der Gleithülse möglich ist und somit die Operationsmethode vereinfacht und verkürzt wird;
- das laterale Gleiten des longitudinalen Knochenfixationselementes wird durch die Blockiermittel nicht limitiert;
- der vorzugsweise als Schraube oder Helixklinge ausgebildete Vorderteil des longitudinalen Knochenfixationselementes sich optimal in der Spongiosa des Femurkopfes verankern kann, weil der Schaft des longitudinalen Knochenfixationselementes in der ihn umschliessenden Hülse während dem Einbringen rotativ frei beweglich bleibt; und
- das longitudinale Knochenfixationselement beim Einbringen in den Femurkopf nicht speziell ausgerichtet werden muss und sich beim Einschlagen helixförmig in den Femurkopf drehen kann. Das longitudinale Knochenfixationselement ist in diesem Zeitpunkt noch nicht gegen Rotation gesichert. Der Chirurg kann somit noch Rotationskorrekturen des Femurkopfes vornehmen, bevor er die Rotation des longitudinalen Knochenfixationselementes in der Längsbohrung der Gleithülse blockiert.

Weitere vorteilhafte Ausgestaltungen der Erfindung sind in den abhängigen Ansprüchen gekennzeichnet.

In einer bevorzugten Ausführungsform umfassen die Blockiermittel eine axialfest und rotativ bewegbar mit dem Schaft verbundene Spannschraube, mittels welcher eine in der Längsbohrung der Gleithülse axial verschiebbare und relativ zur Gleithülse rotativ feste Büchse. Vorzugsweise sind am vorderen Ende der Büchse und am freien Ende des Schaftes miteinander rotativ formschlüssig in Eingriff bringbare Mittel angeordnet.

In einer anderen Ausführungsform sind die Mittel am vorderen Ende der Büchse und am freien Ende des Schaftes als komplementäre Stirnverzahnungen ausgebildet. Damit sind die Vorteile erreichbar, dass
- die Stirnverzahnungen einen Eingriff bei verschiedenen Drehwinkeln zwischen dem Knochenfixationselement und der Gleithülse gestattet, so dass eine einfache Justierung zwischen Knochenfixationselement und intramedullärem Nagel während der Implantation ermöglicht wird; und
- durch die Stirnverzahnungen eine sichere Verdrehsicherung erreichbar ist.

In wiederum einer anderen Ausführungsform weist die Spannschraube an ihrem vorderen Ende mit einem ringförmigen Wulst auf während der Schaft eine koaxiale Bohrung mit einem den Wulst aufnehmenden Hinterstich aufweist, so dass die Spannschraube rotierbar aber axial fest mit dem Schaft verbunden ist. Vorzugsweise umfassen die Bohrung und der Hinterstich eine radiale Öffnung, so dass die Spannschraube quer zur Längsachse des Knochenfixationselementes montierbar ist.

In einer weiteren Ausführungsform ist der unrunde Durchgang komplementär zum unrunden Querschnitt der äusseren Mantelfläche der Gleithülse ausgebildet, wobei beispielsweise der Querschnitt des Durchgangs so ausgestaltet ist, dass der unrunde Querschnitt des Durchgangs periphere Teilabschnitte in Form von Teilkreisbögen aufweist.

Die Fixationsmittel des longitudinalen Knochenfixationselementes bestehen bevorzugt aus einer Helixklinge, vorzugsweise eine Doppelhelixklinge. Andere Ausgestaltungen der Fixationsmittel sind ein Schraubengewinde mit relativ geringer Steigung, ein Meissel, ein Nagel, ein T-Profil oder ein Doppel-T-Profil.

In einer anderen Ausführungsform ist der Kopfteil des longitudinalen Knochenfixationselementes als mehrgängiges Gewinde, vorzugsweise als viergängiges Gewinde ausgebildet. Durch diese Ausgestaltung spielt die Positionierung de Knochenfixationselementes keine Rolle. Das Gewinde des Kopfteils kann dabei eine Steigung von mindestens 50 mm, vorzugsweise mindestens 80 mm aufweisen. Der Vorteil dieser relativ hohen Steigung liegt im höheren Widerstand gegen Rotation des Knochenfixationselementes. Zudem zerstört das als Helixklinge ausgebildete Knochenfixationselement weniger Knochensubstanz als eine konventionelle Hüftschraube mit relativ geringer Gewindesteigung. Der Knochen wird durch die Helixflächen der Helixklinge mehr kompaktiert als geschnitten.

Die Blockiermittel sind vorzugsweise derart dimensioniert, dass sie bezüglich des Durchgangs des intramedullären Nagels als axialer Stop wirken. Dieser Stop vermeidet eine zu weite Migration des Knochenfixationselementes nach medial.

In einer weiteren Ausführungsform ist das longitudinale Knochenfixationselement eine Hüftschraube.

In wiederum einer weiteren Ausführungsform ist das longitudinale Knochenfixationselement eine Helix-Schraube.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Seitenansicht durch die erfindungsgemässe Vorrichtung;
Fig. 2 einen partiellen Schnitt durch einen Marknagel im Bereich seines schrägen Durchgangs mit eingesetzter Hüftschraube und Gleithülse;
Fig. 3 einen partiellen Schnitt durch einen modifizierten Marknagel im Bereich seines schrägen Durchgangs mit eingesetzter Hüftschraube und Gleithülse;
Fig. 4 einen partiellen Schnitt durch eine weitere Modifikation eines Marknagels im Bereich seines schrägen Durchgangs mit eingesetzter Hüftschraube und Gleithülse;
Fig. 5 einen partiellen Schnitt durch die Vorrichtung gemäss Fig. 1;
Fig. 6 einen vergrösserten Ausschnitt des Kreises VI in Fig. 5;
Fig. 7 einen Schnitt längs der Linie VII-VII in Fig. 5;
Fig. 8 einen Längsschnitt durch die Gleithülse mit darin vormontiertem Knochenfixationselement;
Fig. 9a einen vergrösserten Ausschnitt des Kreises VI in Fig. 5 mit blockierten Blockiermitteln;
Fig. 9b einen vergrösserten Ausschnitt des Kreises VI in Fig. 5 mit deblockierten Blockiermitteln;
Fig. 10 einen Längsschnitt durch die in Fig. 2 dargestellte Ausführungsform der Gleithülse;
Fig. 11 eine Ansicht von hinten auf die in Fig. 10 dargestellte Ausführungsform der Gleithülse;
Fig. 12 einen Längsschnitt durch die in Fig. 2 dargestellte Ausführungsform der Gleithülse mit eingeführtem Schaft des Knochenfixationselementes; und
Fig. 13 einen Schnitt längs der Linie III-III in Fig. 12.

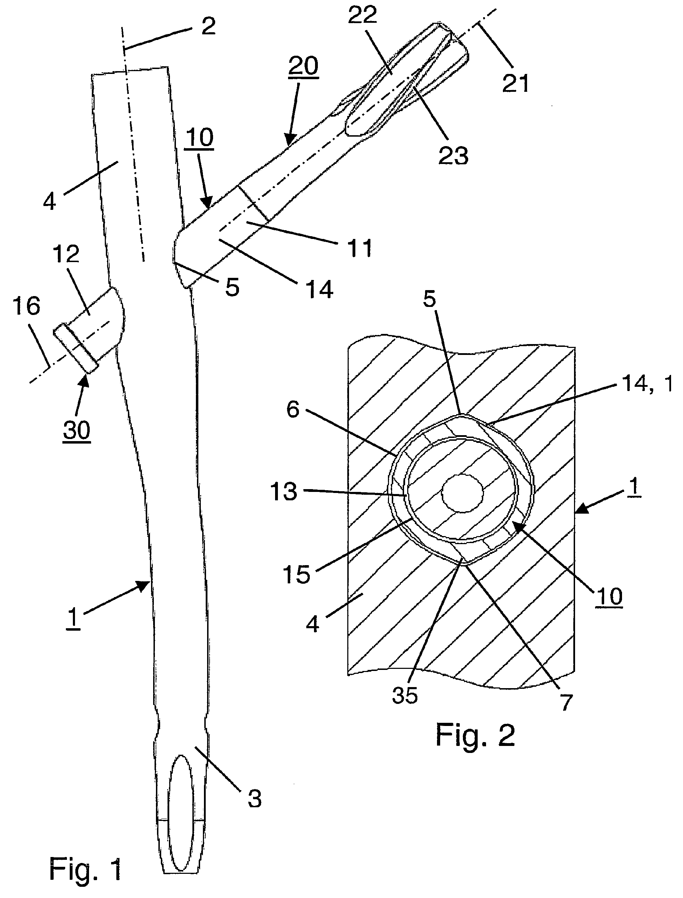

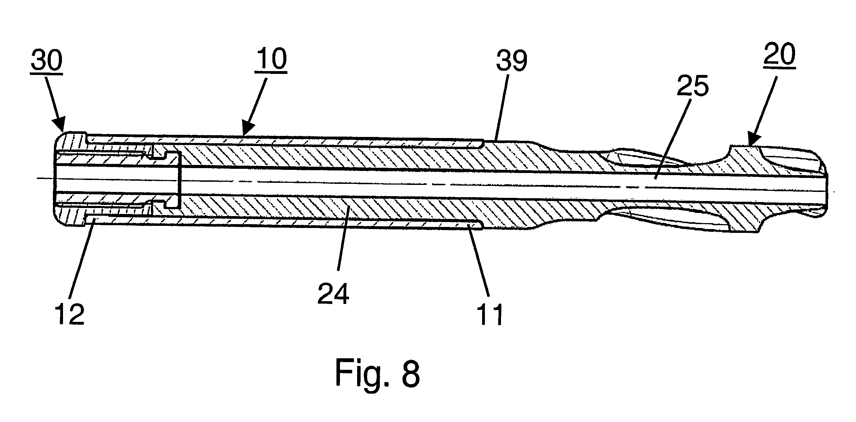

In den Fig. 1 bis 2 sowie 5 bis 8 ist eine Vorrichtung zur Behandlung von Frakturen des Femur dargestellt, welche einen intramedullären Nagel 1, eine Gleithülse 10, ein longitudinales Knochenfixationselement 20 in Form einer Hüftschraube oder Helixschraube und ein Blockiermittel 30 umfasst. Der intramedulläre Nagel 1 besitzt eine zentrale Längsachse 2, einen in den Markkanal des Femur einführbaren Vorderteil 3, einen Hinterteil 4, sowie einen den Hinterteil 4, schräg zur Längsachse 2 durchbohrenden Durchgang 5 mit einem unrunden Querschnitt 6 (Fig. 2 bis 4). Die durch den unrunden Durchgang 5 hindurchführbare Gleithülse 10 besitzt ein vorderes Ende 11, ein hinteres Ende 12, eine zentrale Längsbohrung 13, eine äussere Mantelfläche 14, eine innere Mantelfläche 15 sowie eine Längsachse 16.

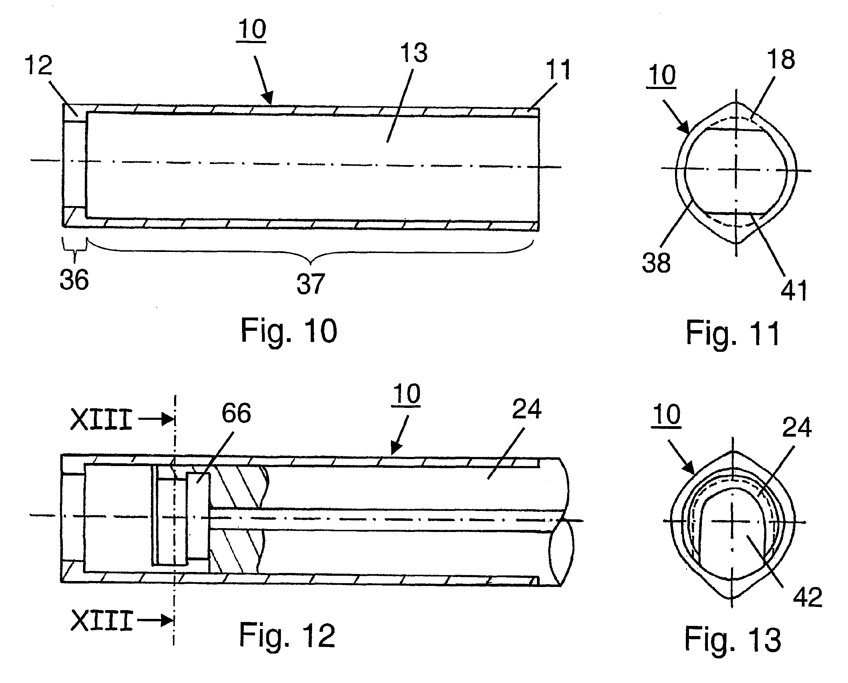

Das longitudinale Knochenfixationselement 20 in Form einer Hüftschraube oder Helixschraube besitzt eine Längsachse 21, einen Kopfteil 22 mit Fixationsmitteln 23, in Form eines mehrgängigen Gewindes relativ hoher Steigung, die bei Benutzung mit dem Femurkopf in Eingriff bringbar sind, sowie einem, koaxial in die Gleithülse 10 einführbaren Schaft 24. Die äussere Mantelfläche 14 der Gleithülse 10 weist einen unrunden Querschnitt 17 auf, während die innere Mantelfläche 15 der Gleithülse 10 nur auf einem an ihr hinteres Ende 12 angrenzenden, hinteren Segment 36 einen unrunden Querschnitt 38 und auf einem vorderen Segment 37 einen runden Querschnitt 18 aufweist (Fig. 10;11). Der runde Querschnitt 18 des vorderen Segmentes 37 (Fig.10; 11) weist denselben Durchmesser wie der Schaft 24 des Knochenfixationselementes 20 auf, so dass das Knochenfixationselement 20 axial verschiebbar und um die Längsachse 16 rotierbar in der Gleithülse 10 gelagert ist. Ein Segment am Übergang zwischen diesem in die Längsbohrung 13 einführbaren Segment des Schaftes 24 und dem Kopfteil 22 des Knochenfixationselementes 20 weist einen grösseren Durchmesser auf, so dass dadurch eine am vorderen Ende 11 der Gleithülse 10 zur Anlage bringbare Schulter 39 gebildet. Durch diese Schulter 39 wird die axiale Verschiebbarkeit des Knochenfixationselementes 20 gegen das hintere Ende 11 der Gleithülse 10 begrenzt. Schliesslich sind Blockiermittel 30 vorgesehen, um die Rotation der Hüftschraube in der Gleithülse 10 wahlweise blockieren zu können.

Wie in Fig. 2 dargestellt wird die Gleithülse 10 durch ihre einen unrunden Querschnitt 17 aufweisende äussere Mantelfläche 14 im ebenfalls einen unrunden Querschnitt 6 aufweisenden Durchgang 5 des Marknagels 1 gegen Rotation gesichert. Der unrunde Querschnitt 6 des Durchgangs 5 ist - wie in Fig. 2 dargestellt - im wesentlichen als Kreis mit zwei diametral angeordneten, zur Längsachse 16 der Gleithülse 10 parallelen Rillen 7 ausgebildet. Durch die dazu komplementäre Ausgestaltung der äusseren Mantelfläche 14 der Gleithülse 10 mit zwei Erhebungen 35 ist die Gleithülse 10 im Durchgang 5 gegen Rotation relativ zum Marknagel 1 gesichert. Dank dieser Gestaltung kann der Marknagel 1 auch mit einer konventionellen Hüftschraube mit kreiszylindrischen Schaft verwendet werden, d.h. auch ohne Gleithülse.

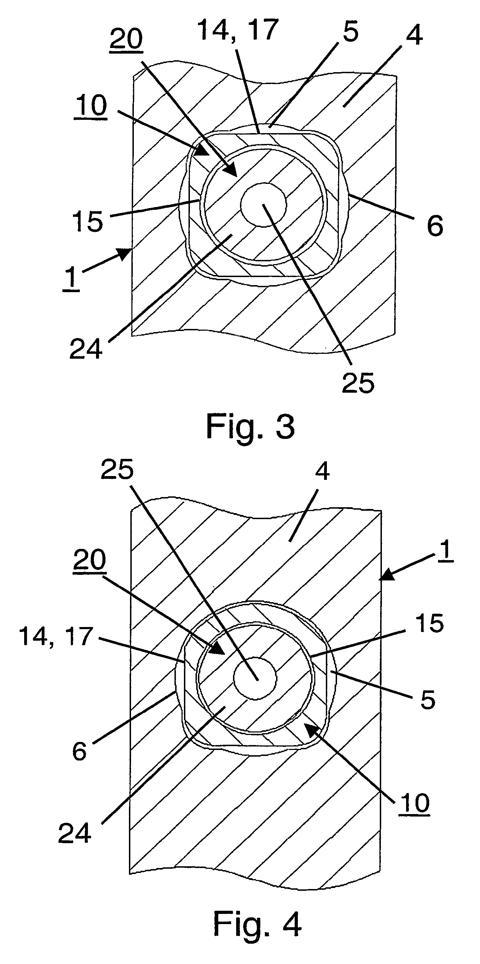

In Fig. 3 ist ein Variante des unrunden Querschnitts 6 des Durchgangs 5 dargestellt. Der kreiszylindrische Schaft 24 des als Hüftschraube ausgebildeten longitudinalen Knochenfixationselementes 20 ist im Inneren der im vorderen Segment 37 eine kreiszylindrische innere Mantelfläche 15 aufweisenden Gleithülse 10 rotativ frei gelagert. Die im wesentlichen einen quadratischen, d.h. unrunden Querschnitt 17 aufweisende äussere Mantelfläche 14 der Gleithülse 10 ist dagegen im ebenfalls einen unrunden Querschnitt 6 aufweisenden Durchgang 5 des Marknagels 1 gegen Rotation gesichert. Der unrunde Querschnitt 6 des Durchgangs 5 ist - wie in Fig. 3 dargestellt - ebenfalls annähernd quadratisch ausgebildet, weist aber zusätzlich periphere Teilabschnitte in Form von Teilkreisbögen auf. Dank dieser Gestaltung kann der Marknagel 1 auch mit einer konventionellen Hüftschraube mit kreiszylindrischen Schaft verwendet werden, d.h. auch ohne Gleithülse.

In Fig. 4 ist eine weitere Variante eines unrunden Querschnitts 6 des Durchgangs 5 dargestellt, bei welchem zwei kleine Teilkreisbögen und einer grösserer Teilkreisbogen vorhanden sind. Entsprechend ist der unrunde Querschnitt 17 der äusseren Mantelfläche 14 der Gleithülse 10 ausgebildet.

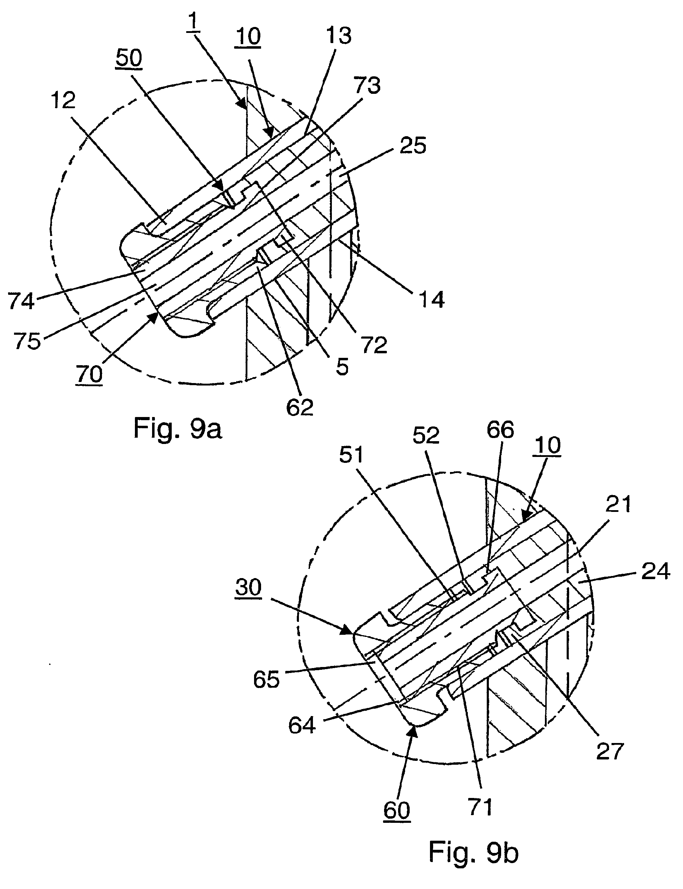

Wie in den Fig. 9a;9b dargestellt umfassen die Blockiermittel 30 eine parallel zur Längsachse 21 des Knochenfixationselementes 20 verschiebbare Büchse 60 sowie eine Spannschraube 70. Die Büchse 60 ist am hinteren Ende 12 der Gleithülse 10 in deren Längsbohrung 13 axial verschiebbar gelagert.

Wie in den Fig. 10 und 12 dargestellt umfasst die Längsbohrung 13 in der Gleithülse 10 zwei axial aneinandergrenzende Segmente 36;37, wovon das vordere Segment 37 einen runden Querschnitt 18 aufweist. Der Schaft 24 des Knochenfixationselementes 20 ist daher bei deblockierten Blockiermitteln 30 (Fig. 9b) um die Längsachse 16 der Gleithülse 10 rotierbar in dieser gelagert.

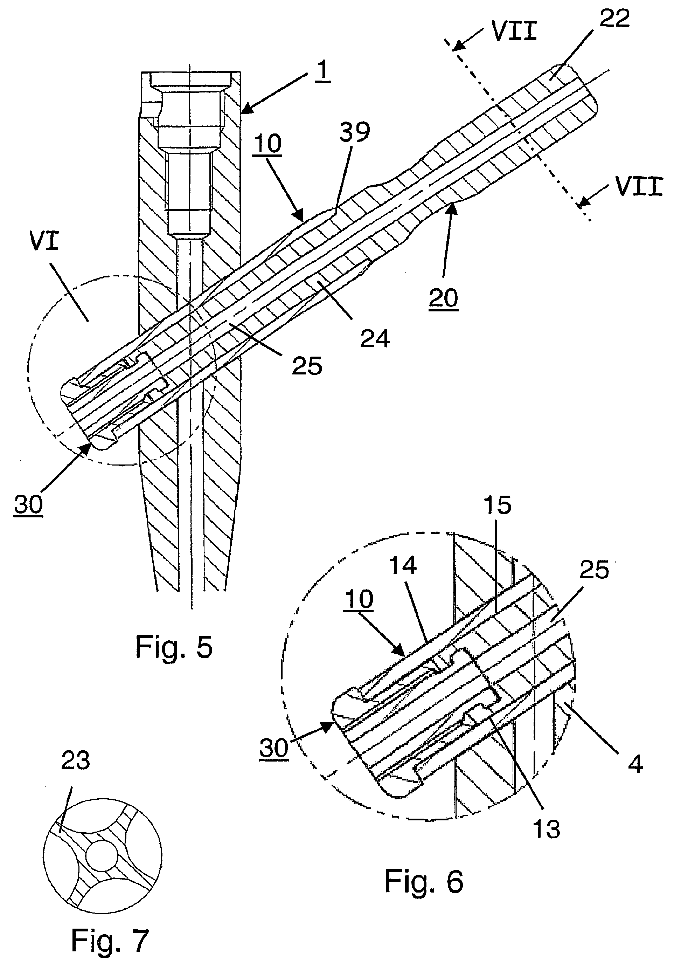

Wie in Fig. 7 dargestellt, sind die Fixationsmittel 23 des longitudinalen Knochenfixationselementes 20 als viergängige Helix-Schraube ausgebildet, wobei das Gewinde eine Steigung von ca. 120 mm aufweist.

Wie in den Fig. 9a und 9b dargestellt sind zwischen dem vorderen Ende 62 der Büchse 60 und dem freien Ende 27 des Schaftes 24 miteinander rotativ formschlüssig in Eingriff bringbare Mittel 50 angeordnet, welche durch axiale Verschiebung der Büchse 60 in Eingriff bringbar sind. Wenn die Mittel 50 in Eingriff, d.h. blockiert sind (Fig. 9a), wird eine relative Rotation zwischen Büchse 60 und Schaft 24 um die Längsachse 21 des Knochenfixationselementes 20 verhindert, während bei deblockierten Mitteln 50 (Fig. 9b) eine Rotation des Knochenfixationselementes 20 relativ zur Gleithülse 10 möglich ist. Die Mittel 50 sind hier als Stirnverzahnungen 51 ;52 ausgebildet, wobei am vorderen Ende 62 der Büchse 60 eine kreisringartige, ersten Stirnverzahnung 51 angeordnet ist und die zweite Stirnverzahnung 52 ebenfalls kreisringartig am freien Ende 27 des Schaftes 24 angeordnet ist. Die Büchse 60 weist eine Zentralbohrung 65 mit einem koaxialen, auf das Aussengewinde 71 der Spannschraube 70 schraubares Innengewinde 64 auf. Beim Anziehen der Spannschraube 70 wird die Büchse 60 axial gegen das freie Ende 27 des Schaftes 24 verschoben bis die zwei Stirnverzahnungen 51;52 am vorderen Ende 62 der Büchse 60 und am freien Ende 27 des Schaftes 24 in Eingriff gebracht sind (Fig. 9a). Dadurch wird zwischen der Büchse 60 und dem Schaft 24 eine relative Rotation um die Längsachse 21 des Knochenfixationselementes 20 verhindert. Die Längsbohrung 13 weist im Bereich ihres hinteren Segmentes 36 einen unrunden Querschnitt 38 mit ebenfalls zwei Abflachungen 41 auf (Fig. 11). Durch die komplementär unrunden Querschnitte der Büchse 60 und der Längsbohrung 13 in der Gleithülse 10 wird eine Rotation zwischen der Büchse 60 und der Gleithülse 10 verhindert.

Bei blockierten Blockiermitteln 30 (Fig. 9a) ist somit einerseits das Knochenfixationselement 20 gegen Rotation relativ zur Gleithülse 10 gesichert und andererseits die Gleithülse 10 wegen der unrunden Querschnitte 6; 17 von Durchgang 5 und äusserer Mantelfläche 14 gegen eine Rotation relativ zum intramedullären Nagel 1 gesichert.

Die Spannschraube 70 ist axial fest und rotativ bewegbar mit dem freien Ende 27 des Schaftes 24 verbunden. Wie in den Fig. 9a und 9b gezeigt ist diese axiale Verbindung durch einen in einen Hinterstich 66 in der Bohrung 25 im Schaft 24 eingreifenden Wulst 72 am vorderen Ende 73 der Spannschraube 70 realisiert. Da sich der Hinterstich 66 über den gesamten Umfang der Bohrung 25 erstreckt, wird die Rotation der Spannschraube 70 relativ zum Schaft 24 um die Längsachse 21 des Knochenfixationselementes 20 nicht behindert. Die Spannschraube 70 ist koaxial durchbohrt und weist an ihrem hinteren Ende 74 Mittel 75 zur Aufnahme eines Schraubendrehers (nicht gezeichnet) auf. Durch die axiale Fixierung der Spannschraube 70 am Schaft 24 wird die Büchse 60 beim Anziehen der Spannschraube 70 gegen das freie Ende 27 des Schaftes 24 bewegt bis die zwei Stirnverzahnungen 51;52 miteinander im Eingriff stehen, während beim Lösen der Spannschraube 70 die Büchse 60 in die Gegenrichtung verschoben wird, bis die zwei Stirnverzahnungen 51;52 ausgerastet sind. Wie aus den Fig. 12 und 13 ersichtlich, weist die Wand des Schaftes 24 im Bereich der des freien Endes 27 des Schaftes 24 eine radiale Öffnung 42 auf. Und zwar so, dass die Spannschraube 70 mit dem Wulst 72 seitlich in den Hinterstich 66 einführbar ist. Dieses seitliche Einführen der Spannschraube 70 in die radiale Öffnung 42 erfolgt vor dem Überschieben der Gleithülse 10 über den Schaft 24. Anschliessend wird die Spannschraube 70 in das Innengewinde 64 der Zentralbohrung 65 in der Büchse 60 eingeschraubt.

## Patentansprüche

1. Vorrichtung zur Behandlung von Frakturen des Femur mit
A) einem intramedullären Nagel (1) mit einer zentralen Längsachse (2), einem in den Markkanal des Femur einführbaren Vorderteil (3), einem Hinterteil (4), einem den Hinterteil (4), schräg zur Längsachse (2) durchbohrenden Durchgang (5) mit unrundem Querschnitt (6);
B) einer durch den unrunden Durchgang (5) hindurchführbaren Gleithülse (10), mit einem vorderen Ende (11), einem hinteren Ende (12), einer zentralen Längsbohrung (13), einer äusseren Mantelfläche (14), einer inneren Mantelfläche (15) und einer Längsachse (16);
C) einem longitudinalen Knochenfixationselement (20) mit einer Längsachse (21), einem Kopfteil (22) mit Fixationsmitteln (23), die bei Benutzung mit dem Femurkopf in Eingriff bringbar sind, sowie einem, koaxial in die Gleithülse (10) einführbaren Schaft (24); wobei
D) die äussere Mantelfläche (14) der Gleithülse (10) mindestens in einem Teilbereich einen unrunden Querschnitt (17) aufweist; und
E) die innere Mantelfläche (15) der Gleithülse (10) mindestens in einem Teilbereich einen unrunden Querschnitt (38) aufweist,
**dadurch gekennzeichnet, dass**
F) der Schaft (24) in der Längsbohrung (13) der Gleithülse (10) rotierbar gelagert ist; und
G) am freien Ende (27) des Schaftes (24) Blockiermittel (30) angeordnet sind, mittels welcher der Schaft (24) wahlweise rotativ formschlüssig mit der Gleithülse (10) verbindbar ist, so dass die Rotation des longitudinalen Knochenfixationselementes (20) relativ zur Gleithülse (10) wahlweise blockierbar oder deblockierbar ist.

2. Vorrichtung nach Anspruch 1, **dadurch gekennzeichnet, dass** die Blockiermittel (30) eine axialfest und rotativ bewegbar mit dem Schaft (24) verbundene Spannschraube (70) und eine mittels der Spannschraube (70) axial verschiebbare und in der Längsbohrung (13) der Gleithülse (10) rotativ feste Büchse (60) umfassen.

3. Vorrichtung nach Anspruch 2, **dadurch gekennzeichnet, dass** die Büchse (60) ein vorderes Ende (62) und der Schaft (24) ein freies Ende (27) haben und dass am vorderen Ende (62) der Büchse (60) und am freien Ende (27) des Schaftes (24) miteinander rotativ formschlüssig in Eingriff bringbare Mittel (50) angeordnet sind.

4. Vorrichtung nach Anspruch 3, **dadurch gekennzeichnet, dass** die Mittel (50) eine erste Stirnverzahnung (51) am vorderen Ende (62) der Büchse (60) und eine zweite Stirnverzahnung (52) am freien Ende (27) des Schaftes (24) umfassen.

5. Vorrichtung nach einem der Ansprüche 2 bis 4, **dadurch gekennzeichnet, dass** die Spannschraube (70) ein vorderes Ende (73) mit einem ringförmigen Wulst (72) aufweist, und dass der Schaft (24) eine koaxiale Bohrung (25) mit einem den Wulst (72) rotierbar aufnehmenden Hinterstich (66) aufweist.

6. Vorrichtung nach Anspruch 5, **dadurch gekennzeichnet, dass** die Bohrung (25) und der Hinterstich (66) eine radiale Öffnung (42) aufweisen, so dass die Spannschraube (70) quer zur Längsachse (21) des Knochenfixationselementes (20) montierbar ist.

7. Vorrichtung nach einem der Ansprüche 1 bis 6, **dadurch gekennzeichnet, dass** der unrunde Durchgang (5) komplementär zum unrunden Querschnitt (17) der äusseren Mantelfläche (14) der Gleithülse ausgebildet ist.

8. Vorrichtung nach Anspruch 7, **dadurch gekennzeichnet, dass** der unrunde Querschnitt (6) des Durchgangs (5) periphere Teilabschnitte in Form von Teilkreisbögen aufweist.

9. Vorrichtung nach Anspruch 7 oder 8, **dadurch gekennzeichnet, dass** die Fixationsmittel (23) des longitudinalen Knochenfixationselementes (20) eine Helixklinge, vorzugsweise eine Doppelhelixklinge sind.

10. Vorrichtung nach Anspruch 7 oder 8, **dadurch gekennzeichnet, dass** die Fixationsmittel (23) ein Schraubengewinde, ein Meissel, ein Nagel, ein T-Profil oder ein Doppel-T-Profil sind.

11. Vorrichtung nach einem der Ansprüche 1 bis 10, **dadurch gekennzeichnet, dass** der Kopfteil (22) des longitudinalen Knochenfixationselementes (20) als mehrgängiges Gewinde, vorzugsweise als viergängiges Gewinde ausgebildet ist.

12. Vorrichtung nach Anspruch 11, **dadurch gekennzeichnet, dass** das Gewinde des Kopfteils (22) eine Steigung von mindestens 50 mm, vorzugsweise mindestens 80 mm aufweist.

13. Vorrichtung nach einem der Ansprüche 7 bis 11, **dadurch gekennzeichnet, dass** die Blockiermittel (30) bezüglich des Durchgangs (5) als axialer Stop wirken.

14. Vorrichtung nach einem der Ansprüche 1 bis 13, **dadurch gekennzeichnet, dass** das longitudinale Knochenfixationselement (20) eine Hüftschraube ist.

15. Vorrichtung nach einem der Ansprüche 1 bis 13, **dadurch gekennzeichnet, dass** das longitudinale Knochenfixationselement (20) eine Helix-Schraube ist.

## Claims

1. Device for treating femoral fractures, with
A) a intramedullary nail (1) with a central longitudinal axis (2), a front portion (3) insertable in the femoral medullary channel, a rear portion (4), and a passage (5) with a noncircular cross-section (6) penetrating the rear portion (4) transversally to the longitudinal axis (2);
B) a sliding sleeve (10) movable through the noncircular passage (5) having a front end (11), a rear end (12), a central longitudinal bore (13), an outer peripheral surface (14), an inner peripheral surface (15) and a longitudinal axis (16);
C) a longitudinal bone fixation element (20) with a longitudinal axis (21), a head portion (22) with fixating means (23) capable of being engaged with the femoral head upon use, and a shaft (24) capable of being coaxially inserted into the sliding sleeve (10); where
D) the outer peripheral surface (14) of the sliding sleeve (10) presents a noncircular cross-section (17) in at least one partial portion; and
E) the inner peripheral surface (15) of the sliding sleeve (10) presents a noncircular cross-section (38) in at least one partial portion,
**characterized in that**
F) the shaft (24) is rotationally supported in the longitudinal bore (13) of the sliding sleeve (10), and
G) locking means (30) are arranged at the free end (27) of the shaft (24), by which the shaft (24) can optionally be connected to the sliding sleeve (10) with a rotationally positive lock, so that the rotation of the longitudinal bone fixation element (20) relative to the sliding sleeve (10) can optionally be locked or unlocked.

2. Device according to claim 1, **characterized in that** the locking means (30) comprise an axially fastened and rotationally movable tightening screw (70) connected to the shaft (24) and a bushing (60) capable of being axially slid by the tightening screw (70) and rotationally fastened in the longitudinal bore (13) of the sliding sleeve (10).

3. Device according to claim 2, **characterized in that** the bushing (60) has a front end (62) and the shaft (24) has a free end (27) and that means (50) capable of being engaged with each other with a rotationally positive lock are arranged at the front end (62) of the bushing (60) and at the free end (27) of the shaft (24).

4. Device according to claim 3, **characterized in that** the means (50) comprise first radial serrations (51) at the front end (62) of the bushing (60) and second radial serrations (52) at the free end (27) of the shaft (24).

5. Device according to one of the claims from 2 to 4, **characterized in that** the tightening screw (70) presents a front end (73) with a ring-shaped bulge (72) and that the shaft (24) presents a coaxial bore (25) with a recess (66) capable of rotationally receiving the bulge (72).

6. Device according to claim 5, **characterized in that** the bore (25) and the recess (66) present a radial opening (42), so that the tightening screw (70) can be mounted transversally to the longitudinal axis (21) of the bone fixation element (20).

7. Device according to one of the claims from 1 to 6, **characterized in that** the noncircular passage (5) is conformed complementary to the noncircular cross-section (17) of the outer peripheral surface (14) of the sliding sleeve.

8. Device according to claim 7, **characterized in that** the noncircular cross-section (6) of the passage (5) presents peripheral partial portions in the form of partial circular arcs.

9. Device according to claim 7 or 8, **characterized in that** the fixating means (23) of the longitudinal bone fixation element (20) are a helical blade, preferably a double helical blade.

10. Device according to claim 7 or 8, **characterized in that** the fixating means (23) are a screw thread, a chisel, a nail, a T-profile or a double T-profile.

11. Device according to one of the claims from 1 to 10, **characterized in that** the head portion (22) of the longitudinal bone fixation element (20) is conformed as a multiple thread, preferably a four-start thread.

12. Device according to claim 11, **characterized in that** the thread of the head portion (22) presents a pitch of at least 50 mm, preferably of at least 80 mm.

13. Device according to one of the claims from 7 to 11, **characterized in that** the locking means (30) act as an axial lock with reference to the passage (5).

14. Device according to one of the claims from 1 to 13, **characterized in that** the longitudinal bone fixation element (20) is a hip screw.

15. Device according to one of the claims from 1 to 13, **characterized in that** the longitudinal bone fixation element (20) is a helical screw.

## Revendications

1. Dispositif pour le traitement de fractures du fémur, comprenant
A) un clou intramédullaire (1) ayant un axe longitudinal central (2), une partie avant (3) pouvant être introduite dans le canal médullaire du fémur, une partie arrière (4), un passage (5) traversant la partie arrière (4) en biais par rapport à l'axe longitudinal (2), ayant une section transversale non-ronde (6) ;
B) une douille coulissante (10) pouvant être insérée dans le passage non-rond (5), comprenant une extrémité avant (11), une extrémité arrière (12), un alésage longitudinal central (13), une enveloppe externe (14), une enveloppe interne (15) et un axe longitudinal (16) ;
C) un élément de fixation osseuse longitudinal (20) ayant un axe longitudinal (21), une partie de tête (22) comprenant des moyens de fixation (23) qui, en conditions d'utilisation, peuvent venir en prise avec la tête fémorale, et une tige (24) pouvant être introduite coaxialement dans la douille coulissante (10) ; dans lequel
D) l'enveloppe externe (14) de la douille coulissante (10) présente, au moins dans une zone partielle, une section transversale non-ronde (17) ; et
E) l'enveloppe interne (15) de la douille coulissante (10) présente, au moins dans une zone partielle, une section transversale non-ronde (38),
**caractérisé en ce que**
F) la tige (24) est logée de manière rotative dans l'alésage longitudinal (13) de la douille coulissante (10) ; et
G) des moyens de blocage (30) sont disposés au niveau de l'extrémité libre (27) de la tige (24), au moyen desquels la tige (24) peut être reliée, si souhaité, par emboîtement rotatif avec la douille coulissante (10), si bien que la rotation de l'élément de fixation osseuse longitudinal (20) par rapport à la douille coulissante (10) peut être bloqué ou débloqué comme souhaité.

2. Dispositif selon la revendication 1, **caractérisé en ce que** les moyens de blocage (30) comprennent une vis de serrage (70) reliée avec la tige (24) de manière fixe sur un axe et mobile en rotation et une fourrure (60) déplaçable axialement au moyen de la vis de serrage (70) et fixe en rotation dans l'alésage longitudinal (13) de la douille coulissante (10).

3. Dispositif selon la revendication 2, **caractérisé en ce que** la fourrure (60) présente une extrémité avant (62) et la tige (24) présente une extrémité libre (27) et **en ce que** des moyens (50) pouvant être mis en prise les uns avec les autres par emboîtement rotatif sont disposés au niveau de l'extrémité avant (62) de la fourrure (60) et au niveau de l'extrémité libre (27) de la tige (24).

4. Dispositif selon la revendication 3, **caractérisé en ce que** les moyens (50) comprennent une première denture frontale (51) au niveau de l'extrémité avant (62) de la fourrure (60) et une deuxième denture frontale (52) au niveau de l'extrémité libre (27) de la tige (24).

5. Dispositif selon l'une quelconque des revendications 2 à 4, **caractérisé en ce que** la vis de serrage (70) présente une extrémité avant (73) pourvue d'un bourrelet annulaire (72), et **en ce que** la tige (24) présente un alésage coaxial (25) comprenant une dépouille (66) logeant le bourrelet (72) de manière rotative.

6. Dispositif selon la revendication 5, **caractérisé en ce que** l'alésage (25) et la dépouille (66) présentent une ouverture radiale (42), si bien que la vis de serrage (70) peut être montée transversalement à l'axe longitudinal (21) de l'élément de fixation osseuse (20).

7. Dispositif selon l'une quelconque des revendications 1 à 6, **caractérisé en ce que** le passage ovalisé (5) est formé de manière complémentaire au diamètre ovalisé (17) de l'enveloppe extérieure (14) de la douille coulissante.

8. Dispositif selon la revendication 7, **caractérisé en ce que** le diamètre ovalisé (6) du passage (5) présente des segments périphériques en forme d'arcs de cercles partiels.

9. Dispositif selon la revendication 7 ou 8, **caractérisé en ce que** les moyens de fixation (23) de l'élément de fixation osseuse longitudinal (20) sont une lame hélicoïdale, de préférence une lame bi-hélicoïdale.

10. Dispositif selon la revendication 7 ou 8, **caractérisé en ce que** les moyens de fixation (23) sont un filet de vis, un trépan, un clou, un profil en T ou un profil en double T.

11. Dispositif selon l'une quelconque des revendications 1 à 10, **caractérisé en ce que** la partie de tête (22) de l'élément de fixation osseuse longitudinal (20) est réalisée sous la forme d'un filetage multiple, de préférence d'un filetage quadruple.

12. Dispositif selon la revendication 11, **caractérisé en ce que** le filetage de la partie de tête (22) présente un pas d'au moins 50 mm, de préférence d'au moins 80 mm.

13. Dispositif selon l'une quelconque des revendications 7 à 11, **caractérisé en ce que** les moyens de blocage (30) fonctionnent en tant que blocage axial par rapport au passage (5).

14. Dispositif selon l'une quelconque des revendications 1 à 13, **caractérisé en ce que** l'élément de fixation osseuse longitudinal (20) est une vis de hanche.

15. Dispositif selon l'une quelconque des revendications 1 à 13, **caractérisé en ce que** l'élément de fixation osseuse longitudinal (20) est une vis hélicoïdale.
